# EUROPEAN PATENT APPLICATION

(11) **EP 2 194 048 A1**
(43) Date of publication of application: **09.06.2010**
(21) Application number: 08170435.5
(22) Date of filing: 02.12.2008
(51) Int. Cl.: C07D 257/04, C07D 401/12, A61K 31/41, A61P 9/00

(54) **Nitrate esters for the treatment of vascular and metabolic diseases**

(71) Applicant: Sartor, Dirk, 64668 Rimbach (DE)
(72) Inventor: Sartor, Dirk, 64668 Rimbach (DE)
(74) Representative: Becker, Konrad

(57) **Abstract**

Nitrate esters of valsartan and cilostazol are described. They have valuable properties in the treatment of vascular and metabolic diseases.

## Description

### Field of the invention

The invention relates to nitrate esters of medicinal compounds useful in the treatment of vascular and metabolic diseases.

### Background of the invention

Vascular and metabolic diseases are, despite cancer, the leading causes of death in the Western World. Although many different ways of treating vascular and metabolic diseases are known, there is still a need for improved medication. Life-style modifications and drug therapy can decrease and delay the morbidity and mortality associated with these diseases. Treatments which have been proven to reduce the risk for morbidity and mortality in vascular diseases have been typically shown to either improve impaired vascular function or to delay/prevent the progression of vascular dysfunction caused by hypertension, atherosclerosis or other classical metabolic risk factors. Examples for such treatments are calcium channel blockers, angiotension-enzyme converting inhibitors or angiotension receptor blockers.

In patients with coronary artery disease (CAD) due to atherosclerosis, who are suffering from angina pectoris, one of the established standard treatments involves treatment with organic nitrates, specifically nitrate esters, such as glyceryl trinitrate (nitroglycerine), isosorbide dinitrate, or pentaerythrityl tetranitrate, which act all as coronary vasodilators and improve symptoms and exercise tolerance. Most organic nitrates (e.g. mononitrates and trinitrates) are fast acting pharmaceuticals with a relatively short halflife and have the typical disadvantage that patients develop a nitrate tolerance, meaning that part of the pharmacodynamic effect is lost during chronic treatment and a three times daily dosing regimen.

In the case of peripheral arterial disease (PAD) which is typically caused by hypertension and atherosclerosis and presents clinically with intermittent claudication, compounds with vasodilating properties have been shown to improve symptoms and walking. Two established licensed compounds for treatment of PAD patients are cilostazol (a phosphodiesterase III inhibitor) and pentoxifylline. Cilostazol (U.S. Patent 4,277,479) acts as a direct arterial vasodilator. In addition to its reported vasodilator and antiplatelet effects, cilostazol has been proposed to have beneficial effects on plasma lipoproteins, increasing plasma high density lipoprotein cholesterol and apolipoprotein.

Another group of important pharmaceuticals for the treatment of vascular and metabolic diseases are angiotensin II receptor blockers. The antihypertensive activity is due mainly to selective blockade of AT1 receptors and the consequent reduced pressor effect of angiotensin II. Angiotensin II causes potent vasoconstriction, aldosterone secretion and sympathetic activation. All of these actions contribute to the development of hypertension. There are several marketed compounds of this class, in particular losartan, valsartan, olmesartan, irbesartan, candesartan and telmisartan. With the exception of telmisartan, they all comprise a tetrazole structural unit. Several clinical trials have demonstrated that angiotensin II receptor antagonists are as effective as calcium-channel blockers, beta-blockers, and ACE inhibitors in the treatment of hypertension and induce fewer adverse effects. Valsartan is first described in U.S. Patent 5,399,578.

Nitrate esters of drugs in general are described in WO 00/61357. Nitrate esters of particular angiotensin II receptor blockers are described in WO 2005/011646.

### Summary of the invention

The invention relates to compounds of formula **1A, 1B** or **2** 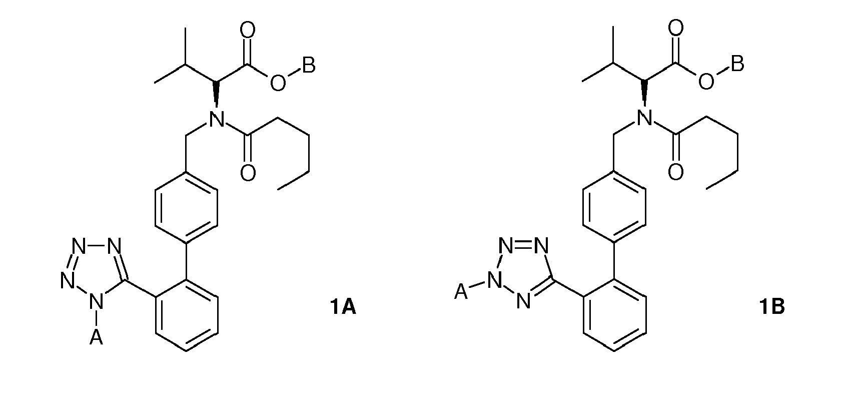 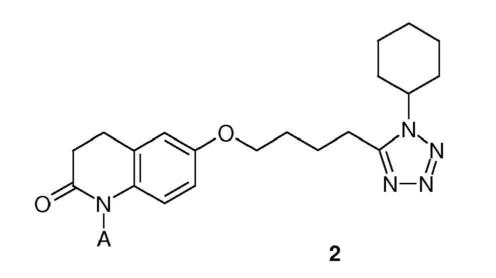
wherein A is
   -(C=O)ₐ-(CH₂)_{b}-O-NO₂;
   -(C=O)-(CH₂OCH₂)_{c}CH₂-O-NO₂;
   -(CH₂CH₂O)_{c}CH₂CH₂-O-NO₂; or
   -(C=O)ₐ-(CH₂)_{d}-CH[(CH₂)ₑ-O-NO₂]₂; and
   a is 0 or 1;
   b is between 1 and 10;
   c is 1, 2 or 3;
   d is 0, 1 or 2; and
   e is between 1 and 4; and
B is
   hydrogen;
   -(CH₂)_{b}-O-NO₂;
   -(CH₂CH₂O)_{c}CH₂CH₂-O-NO₂; or
   -(CH₂)_{d}-CH[(CH₂)ₑ-O-NO₂]₂; and
   b, c, d and e, independent of each other, have the meanings given under A; and
pharmaceutically acceptable salts thereof.

Furthermore the invention relates to pharmaceutical compositions comprising the compounds as defined hereinbefore, to the compounds as defined hereinbefore for the treatment of vascular and metabolic diseases, and to a method of treatment of vascular and metabolic diseases using the compounds and pharmaceutical compositions as defined hereinbefore.

The compounds of the invention represent combinations of useful medicaments for the treatment of vascular diseases such as hypertension, angina pectoris (AP), peripheral artery disease (PAD) and cerebrovascular diseases and have valuable properties in the treatment of metabolic diseases such diabetes and dyslipidaemias. The compounds of the invention have superior vasodilating properties compared to the basic molecules wherein A and B are hydrogen.

### Detailed description of the invention

The compound of formula **1** wherein A and B are hydrogen is known under the name valsartan. Compounds of formula **1A** and **1B** wherein A is hydrogen are tautomers and are in an equilibrium with each other.

The compound of formula **2** wherein A is hydrogen is known under the name cilostazol.

The compounds of formula **1A, 1B** or **2** wherein A and B have the indicated meaning are useful in the treatment of vascular and metabolic diseases.

Vascular and metabolic diseases considered are, for example, atherosclerosis, in particular connected with hypertension, also ocular and pulmonary hypertension, heart failure, in particular chronic heart failure after a heart attack (myocardial infarction), stroke, angina pectoris, cerebrovascular disease, coronary artery disease, left ventricular dysfunction and hypertrophy, peripheral arterial disease (PAD), in particular intermittent claudication.

If B is hydrogen, salts may be formed with suitable cations, in particular with cations providing pharmaceutically acceptable salts.

A pharmaceutically acceptable salt of a compound of formula **1A** or **1B,** wherein B is hydrogen, is e.g. an alkali salt, e.g. the sodium or potassium salt, an earth alkali salt, e.g. the calcium or magnesium salt, or a salt of a protonated nitrogen base, for example ammonium, trimethylammonium, triethylammonium, 2-hydroxyethylammonium, di(2-hydroxyethyl)ammonium or tri(2-hydroxyethyl)ammonium, piperidinium, pyrrolidinium, thiomorpholinium or morpholinium salt, or a quaternary ammonium salt, for example tetramethylammonium, tetraethylammonium, or 2-hydroxyethyltrimethylammonium salt.

Nitrate esters of valsartan have been described previously. It has, however, now be found that a substituent carrying a nitrate ester function can be attached to the tetrazole structural unit, providing easy access to dinitrates, trinitates and tetranitrates. Valsartan derivatives carrying substituents with nitrate ester functions both at the tetrazole unit and as esters of the carboxyl functional group release nitrogen monoxide in a sequential manner and have improved properties.

Compounds of formula **1A, 1B** or **2** can be manufactured by methods well known in the art. Preferably a compound of formula **1A, 1B** or **2,** wherein A and B are hydrogen, is treated with an acylating compound or an alkylating compound, respectively, further carrying one or two bromine atoms, according to standard procedures well known in the art. In the synthesis of a compound of formula **1A** or **1B,** it is preferable to first introduce the residue B by an esterification reaction, and then alkylate or acylate the tetrazole nitrogen in a second step. If the synthesis of a compound of the invention wherein B is hydrogen is intended, this first esterification step is with a reagent that creates an ester as a protective group easily cleavable after alkylation or acylation of the tetrazole function. In the last step of the preferred synthesis, the bromine is replaced by a nitrate ester function by reaction with silver nitrate.

Preferred are compounds of formula **1A, 1B** or **2,** wherein
A is -(C=O)ₐ-(CH₂)_{b}-O-NO₂; or -(C=O)ₐ-(CH₂)_{d}-CH[(CH₂)ₑ-O-NO₂]₂; and
a is 0 or 1; b is between 1 and 6; d is 0, 1 or 2; and e is 1 or 2;
B is hydrogen; -(CH₂)_{b}-O-NO₂; or -(CH₂)_{d}-CH[(CH₂)ₑ-O-NO₂]₂; and
b, d and e, independent of each other, have the meanings given under A; and pharmaceutical acceptable salts thereof.

More preferred are compounds of formula **1 A, 1 B** or **2,** wherein
A is -(C=O)ₐ-(CH₂)_{b}-O-NO₂; or -(C=O)ₐ-(CH₂)_{d}-CH[(CH₂)ₑ-O-NO₂]₂; and
a is 0 or 1; b is between 1 and 6; d is 0, 1 or 2; and e is 1 or 2; and
B is -(CH₂)_{b}-O-NO₂; or -(CH₂)_{d}-CH[(CH₂)ₑ-O-NO₂]₂; and
b, d and e, independent of each other, have the meanings given under A.

Even more preferred are compounds of formula **1A, 1B** or **2,** wherein
A is -(C=O)ₐ-(CH₂)_{b}-O-NO₂; or -(C=O)ₐ-(CH₂)_{d}-CH[(CH₂)ₑ-O-NO₂]₂; and
a is 1; b is 2, 3, 4 or 5; d is 0 or 1; and e is 1 or 2; and
B is -(CH₂)_{b}-O-NO₂; or -(CH₂)_{d}-CH[(CH₂)ₑ-O-NO₂]₂; and
b, d and e, independent of each other, have the meanings given under A.

Even more preferred are compounds of formula **1A** or **1B,** wherein
A is -(C=O)ₐ-(CH₂)_{b}-O-NO₂; and a is 1; and b is 3, 4 or 5; and
B is -(CH₂)_{b}-O-NO₂; and independent of each other, b is 3, 4 or 5.

Likewise preferred are compounds of formula **2**, wherein
A is -(C=O)ₐ-(CH₂)_{d}-CH[(CH₂)ₑ-O-NO₂]₂; and a is 1; d is 0 or 1; and e is 1 or 2.

Most preferred are the compounds of the Examples.

The present invention relates also to pharmaceutical compositions that comprise a compound of formula **1A, 1B** or **2** as active ingredient and that can be used especially in the treatment of the diseases mentioned above. Compositions for enteral administration, such as nasal, buccal, rectal or, especially, oral administration, and for parenteral administration, such as intravenous, intramuscular or subcutaneous administration, to warm-blooded animals, especially humans, are especially preferred. The compositions comprise the active ingredient alone or, preferably, together with a pharmaceutically acceptable carrier. The dosage of the active ingredient depends upon the disease to be treated and upon the species, its age, weight, and individual condition, the individual pharmacokinetic data, and the mode of administration.

The present invention relates especially to pharmaceutical compositions that comprise a compound of formula **1A, 1B** or **2** or a pharmaceutically acceptable salt of a compound of formula **1A** or **1B,** wherein B is hydrogen, and at least one pharmaceutically acceptable carrier.

The invention relates also to pharmaceutical compositions for use in a method for the prophylactic or especially therapeutic management of the human or animal body, in particular in a method of treating a vascular and metabolic disease, especially those mentioned above.

The invention relates also to processes and to the use of compounds of formula **1A, 1B** or **2** thereof for the preparation of pharmaceutical preparations which comprise compounds of formula **1A, 1B** or **2** as active component (active ingredient).

A pharmaceutical composition for the prophylactic or especially therapeutic management of a vascular and metabolic disease, of a warm-blooded animal, especially a human, comprising a novel compound of formula **1A, 1B** or **2** as active ingredient in a quantity that is prophylactically or especially therapeutically active against the said diseases, is likewise preferred.

The pharmaceutical compositions comprise from approximately 1% to approximately 95% active ingredient, single-dose administration forms comprising in the preferred embodiment from approximately 20% to approximately 90% active ingredient and forms that are not of single-dose type comprising in the preferred embodiment from approximately 5% to approximately 20% active ingredient. Unit dose forms are, for example, coated and uncoated tablets, ampoules, vials, suppositories, or capsules. Further dosage forms are, for example, ointments, creams, pastes, foams, tinctures, lipsticks, drops, sprays, dispersions, etc. Examples are capsules containing from about 0.05 g to about 1.0 g active ingredient.

The pharmaceutical compositions of the present invention are prepared in a manner known *per se*, for example by means of conventional mixing, granulating, coating, dissolving or lyophilizing processes.

Preference is given to the use of solutions of the active ingredient, and also suspensions or dispersions, especially isotonic aqueous solutions, dispersions or suspensions which, for example in the case of lyophilized compositions comprising the active ingredient alone or together with a carrier, for example mannitol, can be made up before use. The pharmaceutical compositions may be sterilized and/or may comprise excipients, for example preservatives, stabilizers, wetting agents and/or emulsifiers, solubilizers, salts for regulating osmotic pressure and/or buffers and are prepared in a manner known *per se*, for example by means of conventional dissolving and lyophilizing processes. The said solutions or suspensions may comprise viscosity-increasing agents, typically sodium carboxymethylcellulose, carboxymethylcellulose, dextran, polyvinylpyrrolidone, or gelatins, or also solubilizers, e.g. Tween 80® (polyoxyethylene(20)sorbitan mono-oleate).

Suspensions in oil comprise as the oil component the vegetable, synthetic, or semisynthetic oils customary for injection purposes. In respect of such, special mention may be made of liquid fatty acid esters that contain as the acid component a long-chained fatty acid having from 8 to 22, especially from 12 to 22, carbon atoms. The alcohol component of these fatty acid esters has a maximum of 6 carbon atoms and is a monovalent or polyvalent, for example a mono-, di- or trivalent, alcohol, especially glycol and glycerol. As mixtures of fatty acid esters, vegetable oils such as cottonseed oil, almond oil, olive oil, castor oil, sesame oil, soybean oil and groundnut oil are especially useful.

The manufacture of injectable preparations is usually carried out under sterile conditions, as is the filling, for example, into ampoules or vials, and the sealing of the containers.

Suitable carriers are especially fillers, such as sugars, for example lactose, saccharose, mannitol or sorbitol, cellulose preparations, and/or calcium phosphates, for example tricalcium phosphate or calcium hydrogen phosphate, and also binders, such as starches, for example corn, wheat, rice or potato starch, methylcellulose, hydroxypropyl methylcellulose, sodium carboxymethylcellulose, and/or polyvinylpyrrolidone, and/or, if desired, disintegrators, such as the above-mentioned starches, also carboxymethyl starch, crosslinked polyvinylpyrrolidone, alginic acid or a salt thereof, such as sodium alginate. Additional excipients are especially flow conditioners and lubricants, for example silicic acid, talc, stearic acid or salts thereof, such as magnesium or calcium stearate, and/or polyethylene glycol, or derivatives thereof.

Tablet cores can be provided with suitable, optionally enteric, coatings through the use of, inter alia, concentrated sugar solutions which may comprise gum arabic, talc, polyvinylpyrrolidone, polyethylene glycol and/or titanium dioxide, or coating solutions in suitable organic solvents or solvent mixtures, or, for the preparation of enteric coatings, solutions of suitable cellulose preparations, such as acetylcellulose phthalate or hydroxypropylmethylcellulose phthalate. Dyes or pigments may be added to the tablets or tablet coatings, for example for identification purposes or to indicate different doses of active ingredient.

Pharmaceutical compositions for oral administration also include hard capsules consisting of gelatin, and also soft, sealed capsules consisting of gelatin and a plasticizer, such as glycerol or sorbitol. The hard capsules may contain the active ingredient in the form of granules, for example in admixture with fillers, such as corn starch, binders, and/or glidants, such as talc or magnesium stearate, and optionally stabilizers. In soft capsules, the active ingredient is preferably dissolved or suspended in suitable liquid excipients, such as fatty oils, paraffin oil or liquid polyethylene glycols or fatty acid esters of ethylene or propylene glycol, to which stabilizers and detergents, for example of the polyoxyethylene sorbitan fatty acid ester type, may also be added.

Pharmaceutical compositions suitable for rectal administration are, for example, suppositories that consist of a combination of the active ingredient and a suppository base. Suitable suppository bases are, for example, natural or synthetic triglycerides, paraffin hydrocarbons, polyethylene glycols or higher alkanols.

For parenteral administration, aqueous solutions of an active ingredient in water-soluble form, for example of a water-soluble salt, or aqueous injection suspensions that contain viscosity-increasing substances, for example sodium carboxymethylcellulose, sorbitol and/or dextran, and, if desired, stabilizers, are especially suitable. The active ingredient, optionally together with excipients, can also be in the form of a lyophilizate and can be made into a solution before parenteral administration by the addition of suitable solvents.

Solutions such as are used, for example, for parenteral administration can also be employed as infusion solutions.

Preferred preservatives are, for example, antioxidants, such as ascorbic acid, or microbicides, such as sorbic acid or benzoic acid.

The present invention relates furthermore to a method for the treatment of a vascular and metabolic disease, which comprises administering a compound of formula **1A, 1B** or **2** or a pharmaceutically acceptable salt thereof, wherein the radicals and symbols have the meanings as defined above for formula **1A, 1B** or **2,** in a quantity effective against said disease, to a warm-blooded animal requiring such treatment. The compounds of formula **1A, 1B** or **2** can be administered as such or especially in the form of pharmaceutical compositions, prophylactically or therapeutically, preferably in an amount effective against the said diseases, to a warm-blooded animal, for example a human, requiring such treatment. In the case of an individual having a bodyweight of about 70 kg the daily dose administered is from approximately 0.05 g to approximately 5 g, preferably from approximately 0.25 g to approximately 1.5 g, of a compound of the present invention.

The present invention relates especially also to the use of a compound of formula **1A, 1B** or a pharmaceutically acceptable salt thereof, as such or in the form of a pharmaceutical formulation with at least one pharmaceutically acceptable carrier for the therapeutic and also prophylactic management of a vascular and metabolic disease, in particular of hypertension and congestive heart failure.

Likewise The present invention relates especially also to the use of a compound of formula 2, as such or in the form of a pharmaceutical formulation with at least one pharmaceutically acceptable carrier for the therapeutic and also prophylactic management of a vascular and metabolic disease, in particular of peripheral arterial disease.

The preferred dose quantity, composition, and preparation of pharmaceutical formulations (medicines) which are to be used in each case are described above.

Furthermore, the invention provides a method for the treatment of a metabolic disease, which comprises administering a compound of formula **1A, 1B** or **2** or a pharmaceutically acceptable salt thereof, wherein the radicals and symbols have the meanings as defined above, in a quantity effective against said disease, to a warm-blooded animal requiring such treatment.

The following Examples serve to illustrate the invention without limiting the invention in its scope.

### Examples

### Example 1: (R)-4-Bromobutyl 2-(N-((2'-(1H-tetrazol-5-yl)biphenyl-4-yl)methyl)pentanamido)-3-methylbutanoate

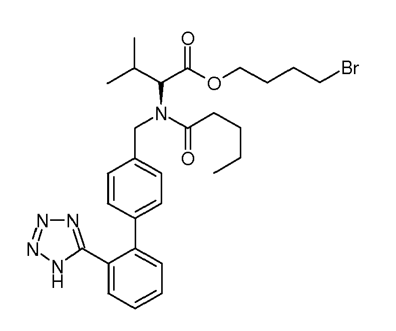

To a solution of (*R*)-2-(*N*-((2'-(1*H*-tetrazol-5-yl)biphenyl-4-yl)methyl)pentanamido)-3-methylbutanoic acid (valsartan) (1.5 g, 3.45 mmol), 4-bromobutan-1-ol (0.65 g, 4.3 mmol) and *N*,*N*-dimethylaminopyridine (0.073 g, 0.6 mmol) in THF (12 ml) cooled to 0°C, dicyclohexylcarbodiimide (1.05 g, 5.1 mmol) is slowly added in portions and the reaction stirred at room temperature for 8 hours. The formed dicyclohexylurea is filtered off and the organic phase is concentrated. The crude material is purified by silica gel chromatography in n-hexane/ethyl acetate 7:3.

### Example 2: (2R)-4-Bromobutyl 2-(N-((2'-(1-(4-bromobutanoyl)-1H-tetrazol-5-yl)biphenyl-4-yl)methyl)pentanamido)-3-methylbutanoate

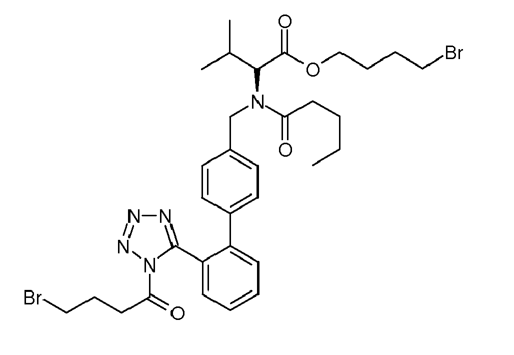

To a solution of (R)-4-bromobutyl 2-(*N*-((2'-(1*H*-tetrazol-5-yl)biphenyl-4-yl)methyl)pentanamido)-3-methylbutanoate (Example 1, 2.0 g, 3.50 mmol), *N*,*N*-dimethylaminopyridine (0.043 g, 0.35 mmol) and triethylamine (0.5 ml, 0.35 mmol) in THF (100 ml) cooled to 0°C and under nitrogen a solution of 4-bromobutanoyl chloride (0.65 g, 3.50 mmol) in THF (5 ml) is slowly added and the reaction stirred at room temperature for 2 hours. Then it is partitioned between ethyl acetate and phosphate buffer (pH = 3) and extracted with ethyl acetate (3 x 25 ml). The organic phase is dried over Na₂SO₄ and concentrated. The crude material is purified by flash chromatography (CH₂Cl₂/acetone 7:3).

### Example 3: (2R)-4-(Nitrooxy)butyl 3-methyl-2-(N-((2'-(1-(4-(nitrooxy)butanoyl)-1H-tetrazol-5-yl)biphenyl-4-yl)methyl)pentanamido)butanoate

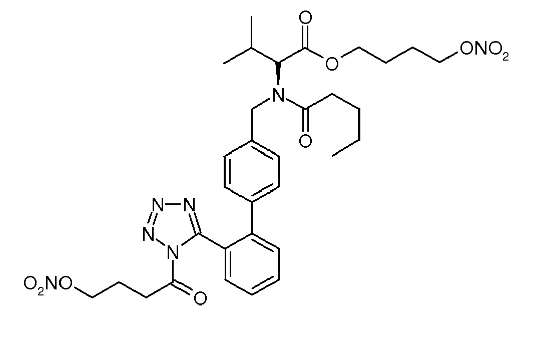

(2R)-4-Bromobutyl 2-(*N*-((2'-(1-(4-bromobutanoyl)-1*H*-tetrazol-5-yl)biphenyl-4-yl)methyl)-pentanamido)-3-methylbutanoate (Example 2, 1.0 g, 1.36 mmol) is dissolved in CH₃CN (20 ml) and AgNO₃ (0.5 g, 3.0 mmol) is added in the dark and under nitrogen. The mixture is stirred at 75°C for 12 hours. Then it is cooled and poured into a phosphate buffer solution (pH = 3). Solid sodium chloride is added and the mixture is extracted with ethyl acetate. The organic phase is washed with phosphate buffer (pH = 3, 1 x 25 ml), brine (3 x 50 ml), dried over Na₂SO₄ and concentrated. The crude material is purified by flash chromatography (CH₂Cl₂/acetone 7:3) affording crude compound, which is dissolved in H₂O/CH₃CN and freeze dried to give the desired dinitrate.

### Example 4: 1-(4-Bromo-3-(bromomethyl)butanoyl)-6-(4-(1-cyclohexyl-1H-tetrazol-5-yl)butoxy)-3,4-dihydroquinolin-2(1H)-one

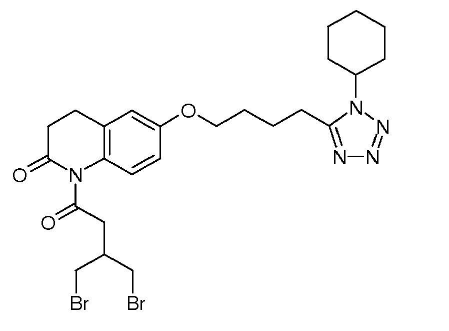

To a solution of (6-(4-(1-cyclohexyl-1*H-*tetrazol-5-yl)butoxy)-3,4-dihydroquinolin-2(1*H*)-one (Cilostazol) (2.0 g, 3.50 mmol), *N*,*N*-dimethylaminopyridine (0.043 g, 0.35 mmol) and triethylamine (0.5 ml, 0.35 mmol) in THF (100 ml) cooled to 0°C and under nitrogen, a solution of 4-bromo-3-(bromomethyl)butanoyl chloride (0.97 g, 3.50 mmol) in THF (5 ml) is slowly and the reaction stirred at room temperature for 2 hours. Then it is partitioned between ethyl acetate and phosphate buffer (pH = 3) and extracted with ethyl acetate (3 x 25 ml). The organic phase is dried over Na₂SO₄ and concentrated. The crude material is purified by flash chromatography (CH₂Cl₂/acetone 7:3).

### Example 5: (2-(2-(6-(4-(1-Cyclohexyl-1H-tetrazol-5-yl)butoxy)-2-oxo-3,4-dihydroqulnoline-2-oxoethyl)propane-1,3-diyl dinitrate

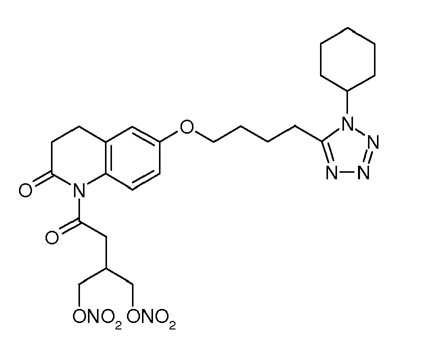

1-(3-Bromo-2-(bromomethyl)butanoyl)-6-(4-(1-cyclohexyl-1*H*-tetrazol-5-yl)butoxy)-3,4-dihydroquinolin-2(1*H*)-one(1.5 g, 2.5 mmol) is dissolved in CH₃CN (30 ml), and AgNO₃ (0.93 g, 5.5 mmol) is added in the dark and under nitrogen. The mixture is stirred at 85°C for 24 hours. Then it is cooled and poured into a phosphate buffer solution (pH = 3). Solid sodium chloride is added and the mixture is extracted with ethyl acetate. The organic phase is washed with phosphate buffer (pH = 3, 1 x 25 ml), brine (3 x 50 ml), dried over Na₂SO₄ and concentrated. The crude material is purified by flash chromatography (CH₂Cl₂/acetone 8:2) affording crude compound, which is dissolved in H₂O/CH₃CN and freeze dried to give the desired dinitrate.

## Claims

1. A compound of formula **1A, 1B** or **2** 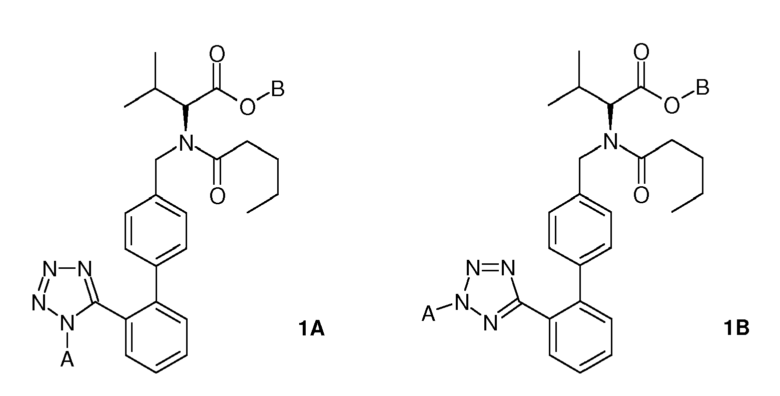 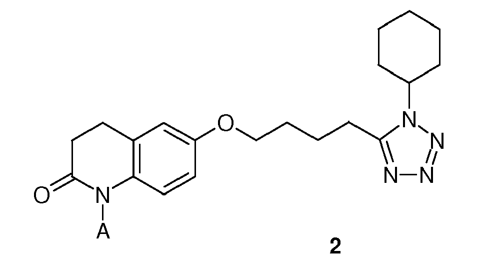
wherein A is
-(C=O)ₐ-(CH₂)_{b}-O-NO_{2;}
-(C=O)-(CH₂OCH₂)_{c}CH₂-O-NO₂;
-(CH₂CH₂O)_{c}CH₂CH₂-O-NO₂; or
-(C=O)ₐ-(CH₂)_{d}-CH[(CH₂)ₑ-O-NO₂]₂; and
a is 0 or 1;
b is between 1 and 10;
c is 1, 2 or 3;
d is 0, 1 or 2; and
e is between 1 and 4; and
B is
hydrogen;
-(CH₂)_{b}-O-NO₂,
-(CH₂CH₂O)_{c}CH₂CH₂-O-NO₂; or
-(CH₂)_{d}-CH[(CH₂)ₑ-O-NO₂]₂; and
b, c, d and e, independent of each other, have the meanings given under A; or
a pharmaceutically acceptable salt thereof.

2. A compound according to claim 1 of formula **1A, 1B** or **2,** wherein
A is -(C=O)ₐ-(CH₂)_{b}-O-NO₂; or -(C=O)ₐ-(CH₂)_{d}-CH[(CH₂)ₑ-O-NO₂]₂; and
a is 0 or 1; b is between 1 and 6; d is 0, 1 or 2; and e is 1 or 2;
B is hydrogen; -(CH₂)_{b}-O-NO₂; or -(CH₂)_{d}-CH[(CH₂)ₑ-O-NO₂]₂; and
b, d and e, independent of each other, have the meanings given under A; and pharmaceutical acceptable salts thereof.

3. A compound according to claim 1 of formula **1A, 1B** or **2,** wherein
A is -(C=O)ₐ-(CH₂)_{b}-O-NO₂; or -(C=O)ₐ-(CH₂)_{d}-CH[(CH₂)ₑ-O-NO₂]₂; and
a is 0 or 1; b is between 1 and 6; d is 0, 1 or 2; and e is 1 or 2; and
B is -(CH₂)_{b}-O-NO₂; or -(CH₂)_{d}-CH[(CH₂)ₑ-O-NO₂]₂; and
b, d and e, independent of each other, have the meanings given under A.

4. A compound according to claim 1 of formula **1A, 1B** or **2,** wherein
A is -(C=O)ₐ-(CH₂)_{b}-O-NO₂; or -(C=O)ₐ-(CH₂)_{d}-CH[(CH₂)ₑ-O-NO₂]₂; and
a is 1; b is 2, 3, 4 or 5; d is 0 or 1; and e is 1 or 2; and
B is -(CH₂)_{b}-O-NO₂; or -(CH₂)_{d}-CH[(CH₂)ₑ-O-NO₂]₂; and
b, d and e, independent of each other, have the meanings given under A.

5. A compound according to claim 1 of formula **1A** or 1 **B,** wherein
A is -(C=0)ₐ-(CH₂)_{b}-O-NO₂; and a is 1; and b is 3, 4 or 5; and
B is -(CH₂)_{b}-O-NO₂; and independent of each other, b is 3, 4 or 5.

6. A compound according to claim 1 of formula **2,** wherein
A is -(C=O)ₐ-(CH₂)_{d}-CH[(CH₂)ₑ-O-NO₂]₂; and a is 1; d is 0 or 1; and e is 1 or 2.

7. A compound according to claim 1 of formula **1A,** wherein
A is -(C=O)-(CH₂)₃-O-NO₂ and B is -(CH₂)₄-O-NO₂.

8. A compound according to claim 1 of formula **2,** wherein
A is -(C=O)-CH₂-CH[CH₂-O-NO₂]₂.

9. A pharmaceutical composition comprising a compound according to any one of claims 1 to 8.

10. A compound according to any one of claims 1 to 8 for the treatment of vascular and metabolic diseases.

11. A method of treatment of vascular and metabolic diseases, which comprises administering a compound according to any one of claims 1 to 8, wherein the radicals and symbols have the meanings as defined above, in a quantity effective against said disease, to a warm-blooded animal requiring such treatment.
